# EUROPEAN PATENT APPLICATION

(11) **EP 3 730 627 A1**
(43) Date of publication of application: **28.10.2020**
(21) Application number: 19170975.7
(22) Date of filing: 25.04.2019
(51) Int. Cl.: C12Q 1/6806, C12N 15/10

(54) **METHOD FOR DONOR IDENTITY VERIFICATION**

(71) Applicant: Kiadis Pharma Intellectual Property BV, 1105 BP Amsterdam (NL)
(72) Inventor: Noordman, Yvet, 1105 BP Amsterdam (NL); Wittmann, Malte, 1105 BP Amsterdam (NL)
(74) Representative: Ringeling, Patricia Louise

(57) **Abstract**

A method for verification of donor identity in a mixed lymphocyte reaction product comprising cell material from a donor and a recipient, the method comprising (i) providing a mixed lymphocyte reaction (MLR) product comprising cell material from a donor and cell material from a recipient; (ii) subjecting the MLR product to DNase treatment; (iii) subjecting the DNase treated MLR product to DNA profiling and (iv) comparing the DNA profile of the DNase treated MLR product to a reference DNA profile of the donor to confirm or reject that the donor in the mixed lymphocyte reaction is the same as the donor of the reference profile.

## Description

### Field of the invention

The present invention relates to a method for donor identity verification. In particular, it relates to the genetic verification of donor identity in a cell based product.

### Background of the invention

Cell therapy products are more frequently used for curing or preventing disease. As part of the manufacturing process of cell therapy products, cells or tissues from different individuals are handled. There is always a risk of mixing up cells from different individuals or cells meant for different individuals or patients. If mixing up occurs, this could lead to immunological rejection, such as graft versus host disease, and it could potentially be lethal.

Adequate cell identification methods which can be used during manufacturing and for testing the end product are therefore required. JP 2003180662 describes an identification code which has to be mixed in a biological sample for identification.

EP 1 614 744 describes the use of single nucleotide polymorphism (SNPs) to prevent the misoperation of cells or tissue from different patients. However, these methods do not provide a solution for preparations comprising a mixture of cells from two individuals, for example donor and recipient cells.

It would be desirable to have a convenient and reliable method for minimizing the chance of a mix up of cells when handling cell preparations comprising a mixture of cells from different individuals.

### Detailed description

The present invention relates to a method according to claim 1. The advantage of the method is that cellular identity can quickly and conveniently be established in a biological sample containing a mixture of cells from different individuals. For example, the method allows convenient and quick verification that a sample comprising a mixture of donor and recipient cells is of donor origin. This minimizes the chance of mixing up samples during cell therapy preparations and treatment.

The biological sample is a mixed cell preparation which comprises cells from different individuals, for example from donor and recipient. The donor may be a donor of an organ or a tissue, which may be solid or non-solid, or may be a donor of cells or of a cell preparation, which may comprise one single cell type or a mixture of cell types, such as PBMCs, white blood cells or T-lymphocytes (T-cells). The biological sample may for example comprise or consist of peripheral blood, peripheral blood mononuclear cells (PBMCs), monocyte-derived dendritic cells or isolated T-lymphocytes from a donor and cells from a recipient, e.g., peripheral blood mononuclear cells (PBMCs) from a recipient.

The recipient may be a transplantation recipient, such as a patient who has received or will receive a transplantation of a solid or non-solid organ. In one embodiment, the recipient has received or will receive stem cell transplantation, typically allogeneic stem cell transplantation. The stem cell transplantation may be part of the treatment of immunological disorders or blood disorders, which may be inherited, such as blood cancers or blood disorders, including acute myeloid leukemia (AML), acute lymphatic leukemia (ALL), chronic myeloid leukemia (CML), chronic lymphatic leukemia (CLL) Hodgkin or non-Hodgkin lymphoma, multiple myeloma, myelodysplastic syndrome (MDS), sickle cell anaemia, severe aplastic anaemia (SAA), granulomatosis or thalassemia.

In one embodiment, the biological sample is a mixed lymphocyte reaction product. In the present context, a mixed lymphocyte reaction (MLR) refers to an ex vivo reaction wherein cell preparations from two individuals, at least one of them containing T-lymphocytes, are mixed to allow a proliferative response of the T-lymphocytes (also referred to as T- cells) from one individual (the responder cells) to cells from another individual (the stimulator cells). For example, it may be used to see how cells of a donor react to cells of a prospective recipient of donor material. In such a case, the recipient cells are the stimulator cells. It may also be used in a process to make donor material suitable for a recipient.

The MLR may be any type of MLR. The MLR is preferably a one-way MLR, wherein the stimulator cells are treated to render them non-proliferative. Cells may be rendered non-proliferative in any suitable way, for example by interfering with their DNA functionality, such as by gamma irradiation, by treating them with mitomycin C or by fixating the cells, e.g. by formaldehyde or paraformaldehyde. The T-cells for MLR may be may be present in a preparation which comprises or consists of T cells. In one embodiment, the T-cells used in the MLR are present in a peripheral blood mononuclear cell (PBMC) fraction. The person skilled in the art knows how to prepare a PBMC fraction, e.g. by Ficoll®- gradient density centrifugation.

In the MLR product, activated or proliferating responder T- cells (alloreactive T-cells) may have been inactivated or destroyed, for example to make donor material suitable for a recipient. This may involve photodynamic photodynamic treatment, for example as described in EP 1 267 931 or in EP 3 258 968, using a rhodamine derivative, such as TH9042. Such alloreactive T- cell depleted mixed lymphocyte reaction products may be used in methods for treating or preventing graft versus host disease (GvHD), for example after or in combination with haploidentical stem cell transplantation (HSCT), in particular after or in combination with T cell depleted CD34+ HSCT.

To confirm the origin of the biological sample, the DNA in the biological sample is subjected to DNase treatment. The biological sample may be fresh or may have been frozen and thawed before being subjected to DNase treatment. The DNA in the biological sample may be any kind of DNA, for example genomic DNA.

The DNase used may be an endonuclease or an exonuclease. The DNase may be fungal, bacterial, yeast, animal or plant produced. It may be a DNase I or DNase II. It may be isolated from its natural environment or it may be synthesized, for example using recombinant technology. Preferably, the DNase preparation is low in RNase and low in protease. More preferably, the DNase is a DNase which is denoted as RNase and protease free. Such DNases are typically produced in non-animal systems, such as in yeast, and are commercially available, for example from Worthington, Lakewood, USA. In one embodiment, RNase and protease free bovine pancreatic deoxyribonuclease I is used.

DNase treatment is carried out by incubating a biological sample for 15 minutes to one hour with DNase at a concentration in the range of 1 U/ml to 1000 U/ml at a temperature in the range of 15 to 37 degrees C. The skilled person will understand that the lower the temperature, the longer the incubation period required. The incubation with DNAse should preferably be long enough to digest all recipient DNA present in the biological sample or at least 40%, at least 50%, at least 60%, at least 70%, at least 80%, or at least 90% of the recipient DNA present in the biological sample. In one embodiment, the biological sample is incubated for 20 to 40 minutes at 30 to 37 degrees C with 100 to 300 U/ml DNase, where 1 U is defined as amount of enzyme added to 1 mg/ml DNA that causes an increase in absorbance of 0.001 per minute at 260 nm.

After DNase treatment, the DNase is inactivated and the DNA profile of the DNase treated biological sample is determined. Any method known in the art may be used for DNase inactivation, such as heat inactivation, EDTA chelation or proteinase K treatment followed by organic extraction. DNA extraction for DNA profiling may be performed using a commercial kit. High quality kits for DNA extraction are commercially available, for example Qiagen DNeasy Blood and Tissue Kit (Qiagen, Hilden, Germany). DNA profiling or genotyping may be performed by any a method available in the art, such as Short Tandem Repeat (STR) profiling, deep sequencing, restriction fragment length polymorphism identification or HLA-typing. Digesting 40% to 100% of the recipient DNA, such as about 50% to 75% or about 60% to 70% of the recipient DNA, will suffice to get good results.

Genomic DNA may conveniently be subjected to STR profiling. STR profiling is based on polymerase chain reaction (PCR) determining polymorphic tetranucleotide or pentanucleotide repeats (Butler JM, J.Forensic Sci. (2006):51:253-65). The varying number of repeats produces DNA amplicons of different sizes, which are identified and assigned a numerical value after comparison to a set of size standards. The pattern of alleles can identify an individual very accurately. For example, using the CODIS 13 core loci the probability of identity is approximately 10⁻¹⁹ (Butler et al., App. Gen. Group, Nat. Inst. of Standards and Technology, Gaithersburg, Maryland, USA, 2012). The advantage of STR profiling over other DNA profiling methods is that it is a quick method which is easy to implement and results are available readily, usually within full DNA digesting is not required for donor confirmation.

STR profiling is preferably performed using standard loci at human genomic DNA. Preferably, at least 5 loci are used, more preferably at least 10 loci are used, such as 10 or more of the core loci commonly used in criminal investigations, viz,. D3S1358, D5S818, D7S820, D8S1179, D13S317, D16S539, D18S51, D21S11, CSF1PO, FGA, TH01, TPOX, vWA and amelogenin, are used for STR profiling. In one embodiment, one or more of Penta D, Penta E, D1S1656, D2S1338, D6S1043, D12S391, D19S433 are used in addition to the core loci. Standard amplification conditions may be used for STR profiling. For efficiency, preferably multiplex PCR is used, allowing simultaneous amplification of several markers. The calling of STR alleles is performed by aligning unknown fragments with a ladder of STR fragments of known allele sizes. STR profiling may be performed using a commercial kit. High quality STR kits are commercially available, for example the Promega PowerPlex 21 Kit with 21 markers (Promega Benelux, Leiden the Netherlands) or the GlobalFiler PCR Amplification Kit with 24 markers (D3S1358, vWA, D16S539, CSF1PO, TPOX, D8S1179, D21S11, D18S51, DYS391, D2S441, D19S433, TH01, FGA, D22S1045, D5S818, D13S317, D7S820, SE33, D10S1248, D1S1656, D12S391, D2S1338, and the sex-determining markers, Y indel and Amelogenin (ThermoFisher Scientific, the Netherlands). For donor identity confirmation using STR profiling, between 5 and 30 or 10 and 30 loci may be used.

The skilled person will understand that any time during processing, genotyping may also be used to verify donor or recipient cell identity, for example to establish a reference STR profile to confirm or reject that the profile of the cell mixture is from the donor.

A DNase treated biological sample comprising cells from different individuals obtained by the method is also part of the invention. It may be used in a manufacturing process of a cell therapy product. For example, it may be used as a means of verification in a method for preventing graft versus host disease, in particular for confirming the cellular identity or origin of a post MLR reaction mixture. Such use is also part of the invention and minimises the chance that patients are infused with their own cells or that cells intended for one patient are infused in another patient. It may be used to confirm the identity at the start of processing, during processing and in the end product. The method according to the invention therefore provides for a safety measure which may be applied in any step of the manufacturing process of a cell therapy product.

### Examples

### Materials and Methods

### Mixed lymphocyte reaction (MLR), Coloration and Photodynamic treatment (PDT)

The mixed lymphocyte reaction (MLR), coloration and Photodynamic treatment (PDT) is performed as described in EP 3 258 968.

In brief, mononuclear cells from a donor and a recipient were isolated by a Ficoll-gradient density centrifugation. Next, the mononuclear cells from the healthy donor were mixed with γ-irradiated mononuclear cells from the recipient in a 1:1 ratio, based on the number of cells. MLR cells are incubated in T-175 flasks at 37°C, 5% CO2. After four days, the post-MLR cells were centrifuged. The resulting pellets were pooled and resuspended in culture medium. The post-MLR cell mixture at a concentration of approximately 1×10⁶ viable cells/mL was mixed in a 1:10 ratio with the dibromorhodamine derivative TH9402 (Kiadis Pharma, the Netherlands), which is 2-(4, 5-dibromo-6-amino-3-imino-3H-xanthen-9-yl) - benzoic acid methyl ester. This coloration mixture was incubated in T-175 flasks at 37°C, 5% CO2. After 40 minutes of coloration, cells were harvested and centrifuged to remove the TH9402-containing medium. Next, the cell pellet was resuspended in extrusion medium (X-VIVO 15™ medium without phenol red (Lonza, USA) and 10% v/v heat-inactivated donor plasma). The cells in extrusion medium were transferred to T-175 flasks. After 90 minutes of extrusion, the flasks containing the cells in extrusion medium were immediately subjected to photodynamic therapy. For photodynamic treatment (PDT), the flasks containing the cells in extrusion medium were placed on an illumination device for photodynamic treatment (Theralux® device Kiadis Pharma, the Netherlands) in a flat position and exposed to light with a wavelength of 514 nm while gently shaking. After light exposure, PDT treated post-MLR cells were collected and centrifuged to remove the extrusion medium. Cell pellets were resuspended in medium.

### Storage of samples

PDT treated post-MLR cells were used fresh, i.e., directly processed for analysis as described in section below, or cryopreserved in 10% v/v Cryostor CS10 (Biolife Solutions) and stored in the vapour phase of the liquid nitrogen until further use. Mononuclear cells from donor and recipient were always cryopreserved in 10% v/v Cryostor CS10 (Biolife Solutions) and stored in the vapour phase of the liquid nitrogen until further use.

### Sample treatment and DNA extraction

Frozen mononuclear cells from donor and recipient, and PDT treated post-MLR cells were thawed at 37°C and directly diluted in X-VIVO 15™ medium. Per sample a total of 5x10⁶ cells were pelleted, washed with 1xPBS, centrifuged and the cell pellet was used for DNA extraction. Thawed or fresh PDT treated post-MLR cells were resuspended in 1 mL X-VIVO 15™ medium, with or without the addition 160U/mL DNase (Worthington, Lakewood, USA), and incubated at 37°C. After 30 minutes of incubation cells were washed with PBS and centrifuged. The cell pellet was used for DNA extraction. DNA isolation was performed with the Qiagen DNeasy Blood and Tissue Kit (Qiagen, Hilde, Germany) according to the manufacturers protocol and DNA was eluted in 200 uL buffer AE (10 mM Tris-CI, 0.5 mM EDTA; pH 9.0, Qiagen, Hilden, Germany).

### Short Tandem Repeat (STR) profiling

STR profiling was performed using Promega PowerPlex 21 Kit with 21 markers (D3S1358, D5S818, D7S820, D8S1179, D13S317, D16S539, D18S51, D21S11, CSF1PO, FGA, TH01, TPOX and vWA, Penta D, Penta E, D1S1656, D2S1338, D6S1043, D12S391, D19S433 plus Amelogenin for gender identification) or the GlobalFiler PCR Amplification Kit with 24 markers (D3S1358, vWA, D16S539, CSF1PO, TPOX, D8S1179, D21S11, D18S51, DYS391, D2S441, D19S433, TH01, FGA, D22S1045, D5S818, D13S317, D7S820, SE33, D10S1248, D1S1656, D12S391, D2S1338, and the sex-determining markers, Y indel and Amelogenin) using standard PCR amplification conditions. STR profiling service was provided by Eurofins Forensic services (Ebersberg, Germany)

### Data evaluation

For peak identification in the STR profiling the threshold for a significant amplification was set manually at 600 or 1000 relative fluorescent units (RFU). Based on this threshold a full STR profile based on the 21 loci was compiled per sample. The STR profiles of the donor and recipient served as reference profiles. STR profiles of the post-MLR samples were compared to the reference profiles and allele origins were assigned.

### Example 1 Donor confirmation in a fresh mixed cellular product

A mixed lymphocyte reaction (MLR) was performed with mononuclear donor and irradiated-recipient cells. MLR, colouration with TH9402 and photodynamic treatment (PDT) were carried out as described under Materials & Methods.

STR analysis was performed on DNA extracted from fresh post-MLR cells with or without DNase treatment. Mononuclear donor and recipient cells were thawed and used to make STR reference profiles. STR analysis was performed using the GlobalFiler PCR Amplification Kit with 24 markers (ThermoFisher Scientific, the Netherlands). The threshold for a significant amplification was set at 600 or 1000 relative fluorescent units (RFU), depending on the channel. Unique STR profiles were returned from the donor and recipient cells (see Table 1). The non-DNase treated fresh post-MLR sample returned a mixed profile based on allelles originating from donor and/or recipient. For the DNase treated fresh post-MLR sample the majority of returned alleles originated from the donor. This shows that treatment of fresh post-MLR samples with DNase reduces the recipient DNA levels significantly and enables confirmation of the cellular identity in fresh post-MLR samples.

**Table 1: STR profiling of fresh post-MLR samples.**

| **Locus** | **Donor PBMC** | **Fresh post-MLR + DNase** | **Fresh post-MLR -DNase** | **Recipient PBMC** |
|---|---|---|---|---|
| **D3S1358** | 14, 16 | 14, 16 | 14, 16 | 16 |
| **vWA** | 18 | 18 | 16, 18 | 16, 18 |
| **D16S539** | 10, 11 | 10, 11 | 10, 11, 12, 13 | 12, 13 |
| **CSF1PO** | 10, 12 | 10, 12 | 10, 11, 12 | 10, 11 |
| **TPOX** | 8, 11 | 8, 11 | 8,11 | 8, 11 |
| **Y Indel** | 2 | 2 | 2 | 2 |
| **AM** | X, Y | X, Y | X, Y | X, Y |
| **D8S1179** | 10, 14 | 10, 13, 14 | 10, 13, 14 | 13, 14 |
| **D21S11** | 31.2, 32.2 | 31.2, 32.2 | 30, 31.2, 32.2 | 30, 31.2 |
| **D18S51** | 13, 19 | 13, 19 | 12, 13, 15, 19 | 12, 15 |
| **D2S441** | 11, 12 | 10, 11, 12 | 10, 11, 12 | 10 |
| **D19S433** | 12.2, 15.2 | 12.2, 15.2 | 12, 12.2, 13.2, 15.2 | 12, 13.2 |
| **TH01** | 6, 9.3 | 6, 9.3 | 6, 8, 9, 9.3 | 8,9 |
| **FGA** | 20, 25 | 20, 24, 25 | 20, 24, 25 | 20, 24 |
| **D22S1045** | 11, 16 | 11, 16 | 11, 16 | 11, 16 |
| **D5S818** | 9, 12 | 9, 12 | 9, 10, 12 | 10, 12 |

### Example 2 Donor confirmation in a freeze-thawed mixed cellular product

A mixed lymphocyte reaction (MLR) was performed with mononuclear donor and irradiated-recipient cells. MLR, colouration with TH9402 and photodynamic treatment (PDT) were carried out as described under Materials & Methods. Mononuclear donor and recipient cells, and PDT treated post-MLR cells were cryopreserved until DNase treatment followed by DNA extraction. Short Tandem Repeat (STR) analysis was performed on extracted DNA. STR profiles obtained from mononuclear cells of donor and recipient served as a reference for the PDT treated post-MLR samples.

For peak identification in the STR analysis the threshold for a significant amplification was set at 1000 relative fluorescent units (RFU). The results are shown in Table 2. Unique STR profiles were returned from the donor and recipient cells. The DNase treated post-MLR sample returned an STR profile which was identical to the STR profile of the donor cells. The non-DNase treated sample returned a mixed profile based on alleles originating from donor and/or recipient and therefore, obtaining confirmation that the mixed cellular product is of donor origin, is not possible In conclusion, treatment of thawed post-MLR samples with DNase reduces the recipient DNA levels below the peak identification threshold enabling confirmation of the cellular identity in the freeze-thawed post-MLR samples.

**Table 2: STR profiling of freeze-thawed post-MLR samples.**

| **Locus** | **Donor PBMC** | **Freeze-Thawed post-MLR + Dnase** | **Freeze-Thawed post-MLR - Dnase** | **Recipient PBMC** |
|---|---|---|---|---|
| AM | X,Y | X,Y | X,Y | X,Y |
| D3S1358 | 15, 19 | 15, 19 | 15, 18, 19 | 15, 18 |
| D1S1656 | 13, 16 | 13, 16 | 13, 14, 16 | 14, 16.3 |
| D6S1043 | 19, 20 | 19, 20 | 11, 19, 20 | 11, 11 |
| D13S317 | 8, 14 | 8, 14 | 8, 12, 14 | 11, 12 |
| Penta E | 12, 13 | 12, 13 | 12, 13 | 11,13 |
| D16S539 | 9, 12 | 9, 12 | 9, 11, 12, 13 | 11, 13 |
| D18S51 | 10, 12 | 10, 12 | 10, 12, 15, 17 | 15, 17 |
| D2S1338 | 18, 20 | 18, 20 | 18, 20, 23 | 22, 23 |
| CSF1PO | 13, 14 | 13, 14 | 9, 13, 14 | 9, 11 |
| Penta D | 12, 13 | 12, 13 | 9,12,13 | 9,13 |
| TH01 | 6, 9.3 | 6, 9.3 | 6, 7, 9.3 | 6, 7 |
| vWA | 16, 17 | 16, 17 | 16, 17 | 17, 17 |
| D21S11 | 29, 31.2 | 29, 31.2 | 29, 31, 31.2 | 29, 31 |

## Claims

1. A method for verification of donor identity in a mixed lymphocyte reaction product comprising cell material from a donor and a recipient, the method comprising:
i- providing a mixed lymphocyte reaction product comprising cell material from a donor and cell material from a recipient;
ii- subjecting the mixed lymphocyte reaction product to DNase treatment
iii- subjecting the DNase treated mixed lymphocyte reaction product to DNA profiling,
iv- comparing the DNA profile of the DNase treated mixed lymphocyte reaction product to a reference DNA profile of the donor to confirm or reject that the donor in the mixed lymphocyte reaction is the same as the donor of the reference profile.

2. A method according to claim 1, wherein the mixed lymphocyte reaction product provided in step i is the product of a one-way mixed lymphocyte reaction.

3. A method according to claim 1 or 2, wherein the cell material from the recipient comprises non-proliferative T-cells.

4. A method according to claims 1-3, wherein the cell material from the donor comprises peripheral mononuclear blood cells.

5. A method according to claims 1-4, wherein the donor and the recipient are haplo-identical.

6. A method according to claims 1-5, wherein the mixed lymphocyte reaction product which is provided in step i is a mixed lymphocyte reaction product wherein the alloreactive donor T cells have been inactivated or destroyed.

7. A method according to claims 1-6, wherein the mixed lymphocyte reaction product provided in step i was frozen and thawed before being subjected to DNase treatment.

8. A method according to claims 1-7, wherein proteinase and RNase free DNase is used.

9. A method according to claims 1-8, wherein the DNA profiling is short tandem repeat (STR) profiling.

10. A method according to claims 1-9, wherein the DNA is genomic DNA.

11. A method according to claims 1-10, wherein the DNA profiling is performed using 5-30 loci

12. A method according to claims 1-11, wherein the recipient has obtained or will obtain stem cell transplantation.

13. A method according to claim 12, wherein the stem cell transplantation is T cell depleted CD34+ stem cell transplantation.

14. A DNase treated mixed lymphocyte reaction product for verification of donor identity.

15. The use of a DNase treated mixed lymphocyte reaction product according to claim 14 for verification of donor identity in a manufacturing process of a cell therapy product.
